Europäisches Patentamt

European Patent Office

Office européen des brevets

(19)

(11) Numéro de publication: **0 138 664**
**A2**

## (12) DEMANDE DE BREVET EUROPEEN

(21) Numéro de dépôt: **84401823.4**

(22) Date de dépôt: **14.09.84**

(51) Int. Cl.⁴: **G 01 N 1/24, A 61 B 5/02**

(30) Priorité: **14.09.83 FR 8314883**

(43) Date de publication de la demande: **24.04.85**
**Bulletin 85/17**

(84) Etats contractants désignés: **AT BE CH DE GB IT LI LU NL SE**

(71) Demandeur: **IFACAP (INSTITUT DE FORMATION POUR L'AMELIORATION DES CONDITIONS d'ACTIVITES PROFESSIONNELLES), Zone Industrielle - P.B. 259, F-59472 Seclin (FR)**

(72) Inventeur: **Kielczewska, Elzbieta, 4, allée des Charmettes, F-59650 Villeneuve D'Ascq (Nord) (FR)**
Inventeur: **Delhaye, Jean-Noel, 20 bis, rue Roger Bouvry, F-59113 Seclin (Nord) (FR)**

(74) Mandataire: **Lepage, Jean-Pierre, Cabinet Lepage & Aubertin Innovations et Prestations 23/25, rue Nicolas Leblanc, F-59011 Lille Cédex 1 (Nord) (FR)**

(54) **Procédé de prélèvement d'air sur un poste de travail et dispositif de mise en oeuvre.**

(57) L'invention est relative à un procédé de prélèvement d'air ambiant et à un dispositif mettant en oeuvre ce procédé.

Selon l'invention, le procédé, dans lequel on aspire en continu, à l'aide d'une pompe l'air ambiant à proximité des voies respiratoires d'un individu occupant un poste de travail, et dans lequel on filtre l'air prélevé en vue d'analyser les particules présentes dans l'air ambiant, est caractérisé en ce que l'on capte le rythme cardiaque de l'individu et que l'on pilote le débit de la pompe avec un signal modulé par le rythme cardiaque.

L'invention trouvera son application dans le domaine de la lutte contre la pollution atmosphérique.

L'invention est relative à un procédé de prélèvement d'air sur un poste de travail, ainsi qu'à un dispositif mettant en oeuvre ce procédé. Elle trouvera son application dans le domaine de la protection des personnes exposées à la pollution atmosphérique.

Les services chargés de la sécurité du travail contrôlent le degré de pollution atmosphérique sur les postes de travail, en particulier dans l'industrie, en effectuant des prélèvements de l'air respiré par les individus occupant les postes de travail dont on veut contrôler le niveau de pollution. Ces prélèvements sont actuellement réalisés à l'aide d'une pompe autonome portée par l'individu au niveau de la ceinture et reliée à un tube de prélèvement qui débouche à proximité de ses voies respiratoires.

Cette pompe est équipée d'un filtre amovible sur lequel viennent se déposer les poussières et les particules présentes dans l'air respiré par l'individu et lorsque le prélèvement est terminé, le filtre est retiré de la pompe en vue d'effectuer une analyse des produits recueillis tant du point de vue quantitatif que du point de vue qualitatif.

Parallèlement à l'analyse des produits recueillis dans le filtre, on réalise une prise de sang du porteur pour tenter d'établir s'il existe une corrélation entre les composants du sang du porteur et la nature et la quantité des produits recueillis dans le filtre.

Or, actuellement il n'a pas été possible d'établir une corrélation entre les résultats de l'analyse de sang et ceux de l'analyse des produits recueillis sur le filtre du dispositif de prélèvement. Cette absence de corrélation est paradoxale, car elle est en contradiction avec de nombreuses observations qui ont permis d'établir, d'une manière non contestable, que la composition du sang d'un individu était fonction de l'atmosphère dans laquelle il respirait.

Or, les dispositifs de prélèvement actuellement utilisés fonctionnent à débit constant et de ce fait ils ne simulent pas correctement la respiration d'une personne car celle-ci dépend étroitement de ses activités physiques. Plus précisément, le rythme respiratoire d'un individu est en corrélation avec son rythme cardiaque, tous deux étant fonction de l'effort physique fourni dans des positions variables selon les conditions.

Le but de l'invention est de proposer un procédé de prélèvement de l'air qui reproduit le rythme respiratoire de l'individu dont on veut contrôler l'atmosphère environnante. En d'autres termes, on propose un procédé par lequel le volume des débits d'air prélevé soit à chaque instant proportionnel au débit pulmonaire de l'individu. A cet effet, l'invention propose de piloter le fonctionnement de la pompe à l'aide d'un signal dépendant du rythme cardiaque de l'individu.

Le rythme cardiaque pourrait naturellement être capté directement au niveau de la poitrine de la personne à l'aide d'électrodes, comme cela est couramment pratiqué pour réaliser un électrocardiogramme. Toutefois, la disposition de ces électrodes est peu compatible avec une activité physique normale sur un lieu de travail. Aussi, l'invention propose de capter le rythme cardiaque au niveau de lobe de l'une des oreilles, en utilisant les propriétés de transparence de celui-ci.

Pour reproduire plus fidèlement le rythme respiratoire de l'individu, le procédé selon l'invention prend également en considération les phénomènes de colmatage pouvant se produire au niveau du filtre de la pompe. A cet effet, le débit réel de l'air au niveau du filtre sera comparé avec le débit d'air théorique obtenu à partir du rythme cardiaque et l'on réalisera éventuellement des corrections pour maintenir le débit de la pompe à la valeur désirée.

Le procédé de prélèvement d'air ambiant sur un poste de travail dans lequel on aspire en continu à l'aide d'une pompe l'air ambiant à proximité des voies respiratoires de l'individu occupant le poste de travail et on filtre l'air prélevé en vue d'analyser les particules présentes dans l'air ambiant, est caractérisé en ce que l'on capte le rythme cardiaque de l'individu et que l'on pilote le débit de la pompe avec un signal modulé par le rythme cardiaque.

L'invention propose également un dispositif de prélèvement mettant en oeuvre ce procédé. Ce dispositif, comprenant notamment un tube de prélèvement d'air, une pompe d'aspiration actionnée par un moteur, reliée au tube de prélèvement et équipée d'un filtre, est caractérisé par le fait qu'il présente des moyens pour calculer le rythme respiratoire à partir de la fréquence cardiaque mesurée et piloter le moteur de la pompe de façon à obtenir le débit d'air proportionnel au débit pulmonaire.

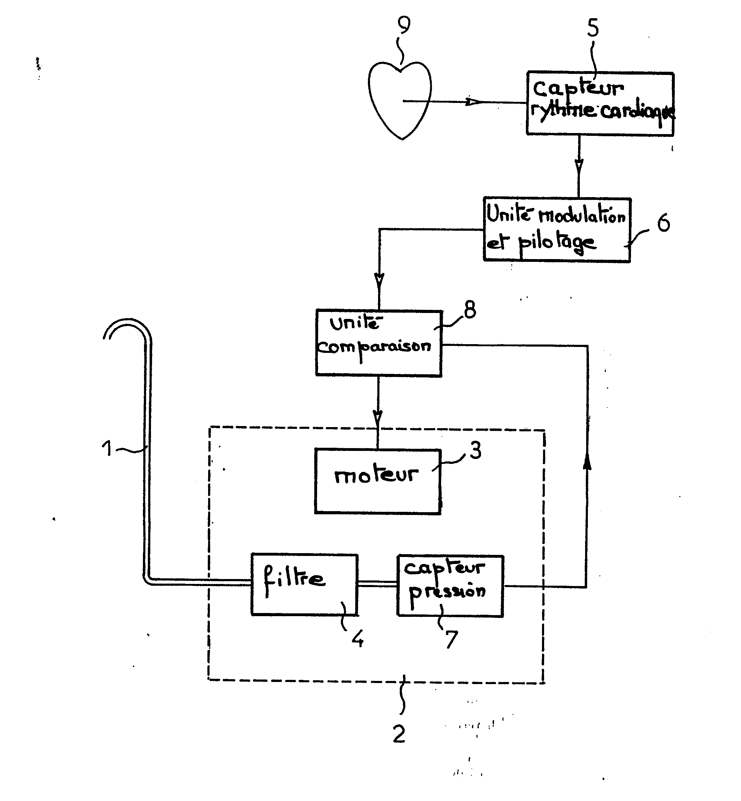

L'invention sera mieux comprise si l'on se réfère à la description ci-dessous ainsi qu'au dessin qui en fait partie intégrante.

La figure unique schématise un mode de réalisation du dispositif mettant en oeuvre le procédé.

L'invention trouvera son application pour tout prélèvement en continu de l'air respiré par un individu. Ce prélèvement pourra être réalisé sur un lieu de travail mais il pourra également être réalisé en tout autre lieu, par exemple, en milieu urbain pour contrôler le niveau de la pollution dûe aux automobiles.

Dans le procédé selon l'invention, on aspire en continu l'air ambiant à proximité des voies respiratoires de l'individu à l'aide d'une pompe d'aspiration conçue pour cette utilisation et dont l'Homme de métier déterminera facilement les caractéristiques. Cette pompe sera portée au niveau de la ceinture et elle présentera une alimentation autonome de façon à ne pas entraver les mouvements du porteur.

En outre, la pompe est équipée d'un filtre qui se présente sous la forme d'une capsule dans laquelle s'accumulent les particules et les poussières présentes dans l'air aspiré.

La caractéristique du procédé selon l'invention est de piloter le fonctionnement de la pompe d'aspiration avec un signal obtenu à partir du rythme cardiaque de l'individu. A cet effet, on capte le rythme cardiaque de façon à produire un signal reproduisant l'effort physique de l'individu, on fait subir à ce signal un traitement afin qu'il puisse piloter le moteur qui actionne la pompe d'aspiration.

Ainsi, les variations de la fréquence cardiaque dûes à l'activité physique entraînent (après le traitement numérique) des variations de la vitesse de la pompe. Cela permet d'accumuler sur le filtre la quantité des poussières proportionnelle à celle qui a pénétré dans l'organisme de l'homme par ses voies respiratoires. Le volume total d'air aspiré par la pompe au cours du prélèvement ainsi que le volume d'air aspiré réellement par l'individu peuvent être lus sur les afficheurs.

Toutefois, pour établir la relation entre les activités cardiaques et respiratoires, il conviendra de tenir compte d'un coefficient propre à chaque individu. Ce coefficient sera déterminé suivant le résultat d'un test que l'on fera subir à l'individu avant d'effectuer les prélèvements de l'air.

Pour tenir compte des phénomènes de colmatage dûs à l'accumulation des poussières sur la surface filtrante, le procédé selon l'invention prévoit que l'on mesure le débit de l'air en aval du filtre, et que ce débit sera comparé avec le débit théorique déterminé suivant le rythme cardiaque. S'il apparaît une différence entre ces deux débits, on effectuera une correction de la tension d'alimentation du moteur de la pompe pour éliminer cet écart. Par conséquent, si le rythme cardiaque traduit un rythme respiratoire constant, le prélèvement de l'air s'effectue également à débit constant.

Par ailleurs, pour faciliter la prise de l'information concernant le rythme cardiaque, celui-ci sera capté directement au niveau du lobe de l'une des oreilles en utilisant la transparence du lobe sous l'action d'un flux lumineux qui est masqué périodiquement par le flux sanguin.

La figure unique illustre un dispositif mettant en oeuvre ce procédé. Ce dispositif comprend tout d'abord un tube de prélèvement 1 dont le type sera facilement déterminé par l'Homme de métier. L'extrémité supérieure de ce tube débouchera au voisinage des voies respiratoires de l'individu et son extrémité inférieure sera reliée à une pompe 2 fixée au niveau de la ceinture du porteur. Cette pompe 2 est actionnée par un moteur 3, relié à un rotor, non représenté, qui permet de créer un mouvement d'aspiration de l'air se trouvant à proximité de l'ouverture supérieure du tube de prélèvement 1. L'air aspiré traverse un filtre 4, présentant une surface filtrante adaptée qui permet de recueillir les fines poussières et les différentes particules véhiculées dans l'air aspiré.

Le dispositif comporte en outre un capteur du rythme cardiaque 5 qui fournit un signal reproduisant le rythme cardiaque. Ce capteur 5 pourra être placé directement à proximité du coeur ou, pour des raisons de commodité, à proximité d'un vaisseau sanguin.

Le signal fourni par le capteur 5 est transmis à une unité de modulation et de pilotage 6 qui transforme un signal de fréquence variable en un signal de tension variable appliqué directement aux bornes du moteur 3 de la pompe. L'unité de modulation devra présenter un temps de réponse très bref étant donné que le signal cardiaque peut atteindre une fréquence de 180 hertz.

Dans un mode préférentiel de réalisation, la pompe sera munie d'un capteur de pression 7 qui permettra de déterminer le débit de l'air aspiré en amont du filtre. L'information concernant ce débit sera transmise à une unité de comparaison 8 dont la fonction sera de déterminer l'écart entre le débit calculé par l'unité de modulation et le débit mesuré à l'aide du capteur de pression différentielle 7. Si l'écart calculé par l'unité de comparaison est négatif, cette unité transmettra une information au moteur de la pompe de façon à accroître sa vitesse et par contre, si l'unité de comparaison détecte un écart positif, elle transmettra au moteur une information pour diminuer le débit de l'air aspiré.

Dans un mode préférentiel de réalisation, le capteur du rythme cardiaque 5 sera disposé au niveau du lobe de l'une des oreilles et il comportera un dispositif permettant d'envoyer un flux lumineux en direction du vaisseau sanguin traversant le lobe et des moyens permettant de mesurer l'intensité lumineuse perçue dans la zone masquée par le vaisseau sanguin si l'on considère la source de lumière.

La réalisation de ce dispositif de prélèvement de l'air ambiant fera appel à des composants électroniques dont la nature, la fonction et la disposition seront déterminées par l'Homme de l'Art.

Le mode de réalisation qui vient d'être décrit n'est donné qu'à titre indicatif et d'autres mises en oeuvre de la présente invention à la portée de l'Homme de l'Art pourraient être adoptées sans pour autant sortir du cadre de celle-ci.

## REVENDICATIONS

1. Procédé de prélèvement d'air ambiant sur un poste de travail dans lequel on aspire en continu à l'aide d'une pompe l'air ambiant à proximité des voies respiratoires de l'individu occupant le poste de travail et on filtre l'air prélevé en vue d'analyser les particules présentes dans l'air ambiant, caractérisé en ce que l'on capte le rythme cardiaque de l'individu et que l'on pilote le débit de la pompe avec un signal modulé par le rythme cardiaque.

2. Procédé selon la revendication 1, caractérisé en ce que l'on mesure le débit d'air prélevé après l'avoir filtré, que l'on compare le débit mesuré avec le débit déterminé par le signal modulé par le rythme cardiaque et que si l'on détecte un écart entre ces deux débits, on agit sur le débit de la pompe pour éliminer cet écart.

3. Procédé selon l'une quelconque des revendications précédentes, caractérisé par le fait que le rythme cardiaque est pris en compte en l'affectant d'un coefficient déterminé par les caractéristiques physiologiques de l'individu.

4. Procédé selon l'une quelconque des revendications précédentes, caractérisé en ce que l'on capte le rythme cardiaque au niveau du lobe de l'une des oreilles de l'individu.

5. Dispositif pour la mise en oeuvre du procédé selon la revendication 1 comprenant notamment un tube de prélèvement (1) de l'air ambiant à proximité des voies respiratoires de l'individu, une pompe d'aspiration (2) actionnée par un moteur (3), reliée au tube de prélèvement (1) et équipée d'un filtre (4) pour recueillir les particules présentes dans l'air prélevé, caractérisé par le fait qu'il présente des moyens pour capter le rythme cardiaque de l'individu et une unité pour moduler le signal engendré par le rythme cardiaque et piloter le moteur (3) de la pompe (1) avec le signal modulé.

6. Dispositif selon la revendication 5, caractérisé par le fait qu'il comprend un capteur (7) pour mesurer le débit de l'air en aval du filtre (4) et une unité (8) pour comparer le débit mesuré par le capteur (7) avec le débit déterminé par le signal modulé par le rythme cardiaque et éventuellement commander le moteur (3) de la pompe pour éliminer cet écart.

7. Dispositif selon la revendication 6, caractérisé par le fait que les moyens pour capter le rythme cardiaque sont disposés au niveau du lobe de l'une des oreilles de l'individu et détectent le rythme

0138664

cardiaque par transparence au moyen d'un flux lumineux dirigé suivant le vaisseau sanguin traversant le lobe de l'oreille considéré.

0138664